# EUROPEAN PATENT APPLICATION

(11) **EP 1 987 829 A1**
(43) Date of publication of application: **05.11.2008**
(21) Application number: 07737295.1
(22) Date of filing: 20.02.2007
(51) Int. Cl.: A61K 31/4188, A61P 7/02, A61P 9/10, C07D 491/107

(54) **PROPHYLACTIC OR THERAPEUTIC AGENT FOR CEREBRAL ISCHEMIA OR CEREBRAL ISCHEMIC REPERFUSION INJURY IN STROKE**

(30) Priority: 20.02.2006 JP 2006041761
(71) Applicant: Sanwa Kagaku Kenkyusho Co., Ltd, Nagoya-shi Aichi 461-8631 (JP)
(72) Inventor: CHUNG, Sookja, Kim, Hong Kong, SAR; (CN); CHUNG, Stephen, Hong Kong, SAR; (CN); HIBI, Chihiro, Nagoya-shi, Aichi 461-8631 (JP)
(74) Representative: Torggler, Paul Norbert
(86) International application number: PCT/JP2007/053036
(87) International publication number: WO 2007/097301

(57) **Abstract**

The present invention provides a preventive or therapeutic agent for cerebral ischemic injury or cerebral ischemic reperfusion injury in stroke such as cerebral infarction and cerebral hemorrhage which exerts its effect through a different mechanism from that of conventional therapeutic agents and can be taken for a long period. The present invention is a preventive or therapeutic agent for cerebral ischemic injury or cerebral ischemic reperfusion injury in stroke which contains 6-fluoro-2',5'-dioxospiro[chroman-4,4'-imidazolidine]-2-carboxamide as an active ingredient. A particularly preferable compound is an optically resolved (2S,4S)-form of fidarestat.

## Description

### TECHNICAL FIELD

The present invention relates to novel medicinal use of 6-fluoro-2',5'-dioxospiro[chroman-4,4'-imidazolidine]-2-carboxamide.

### BACKGROUND ART

The number of patients with acute ischemic stroke is estimated at 370,000, and more cases have arisen because there was an increase in patients with hypertension, those with arteriosclerosis, and those with diabetes. In treatment of stroke such as cerebral infarction and cerebral hemorrhage, it is important how to prevent cerebral injury in acute phase which has a close relation to sequelae. Important factors that limit the prognosis of tissue in the acute phase of cerebral ischaemia are the remaining blood flow and the ischemic interval. Cerebral injury is caused by metabolic disturbance due to the absence of cerebral blood flow, and when the ischemic interval exceeds a certain value, changes in vascular endothelial cells occur, thereby destroying the blood brain barrier and then flowing a large amount of the plasma into the intercellular space. In this state, the brain tissue does not return to normal even if ischemia is reduced, which leads to irreversible cell death and completed infarction. If ischemia is reduced by performing revascularization using a thrombolytic drug in the reversible state, cerebral injury may be prevented.

Then, thrombolytic therapy with therapeutic agents such as tissue plasminogen activator (t-PA) or urokinase (UK) has been performed as the treatment for acute ischemic stroke. However, even if revascularization is successfully performed, reperfusion injury may be induced, and cerebrovascular disorder may be worsened. The administration of large amounts of t-Pa, particularly at advanced stages of vascular endothelial injury frequently causes cerebral hemorrhage complications and exacerbation of cerebral infarction. Therefore, the restriction in which the timing of thrombolytic therapy is within 3 hours from the start of symptoms has been determined. For this reason, there are many problems; for example, only about 5 to 10% of all patients with cerebral infarction can receive t-PA therapy. In order to further widespread revascularization therapy by thrombolytic therapies such as t-PA, an objective is how to reduce reperfusion injury caused by revascularization. In Japan, edaravone which eliminates free radicals for use as a therapeutic agent for cerebral ischemia reperfusion injury has recently been approved for indications of "the improvement of neurological symptoms accompanying acute ischemic stroke and problems with activities of daily living". However, the use of the agent is considerably limited; for example, the agent must be applied within 24 hours after the onset of symptoms and the dosing period is 14 days.

On the other hand, surgical intervention for cerebral infarction, occurred such that cerebral circulation is decreased by lesions in the cervical carotid artery which provides nutrients to the brain tissue or the intracranial main artery and then thrombus is formed at the site of stenosis and necrosis of the brain tissue in the perfusion area is caused by the thrombus, has begun to be performed. Specific examples thereof include carotid artery stent placement, bypass operation, or cervical carotid endarterectomy for cervical carotid artery occlusion or stenotic or obstructive lesion of an intracranial main artery. These surgeries are effective, however, cerebral embolism is caused by tissue fragments and microthrombotic fragments produced by the surgical procedure and it is difficult to completely prevent sequelae of cerebral infarction or reperfusion injury after thrombolysis.

(2S,4S)-6-fluoro-2',5'-dioxospiro[chroman-4,4'-imidazolidine]-2-car boxamide (generic name: Fidarestat) which was found in the present applicant company was developed as a compound having the strong aldose reductase (AR) inhibitory activity and having high safety even when taken over a long period, and currently, a clinical test is being progressed as a therapeutic agent for diabetic neuropathy.

Regarding a hydantoin derivative including fidarestat, use in diabetic neuropathy is described in Japanese Patent Application Laid-Open (JP-A) No. 61-200991, use in various diseases accompanied with aging is described in JP-A No. 6-135968, use in diabetic simple retinopathy is described in JP-A No. 7-242547, use in diabetic keratopathy is described in JP-A No. 8-231549, use in diabetic maculopathy is described in WO2005/072066, and use in severe diabetic retinopathy is described in WO2005/079 Further, use in circulation disease is described in JP-A No. 4-173791. However, fidarestat has no pharmacological effect on the blood coagulation system and the circulatory system as reported in Hatsumei Kyokai Kokai Giho No. 2006-500058. That is, use of fidarestat as a preventive or therapeutic agent for cerebral ischemic injury or cerebral ischemic reperfusion injury has not been reported.
[Patent document 1] JP-A No. 61-200991
[Patent document 2] JP-A No. 6-135968
[Patent document 3] JP-A No. 7-242547
[Patent document 4] JP-A No. 8-231549
[Patent document 5] WO2005/0720
[Patent document 6] WO2005/079792
[Patent document 7] JP-A No. 4-173791
[Nonpatent document 1] Hatsumei Kyokai Kokai Giho No. 2006-500058

### DISCLOSURE OF THE INVENTION

### Problems to be solved by the Invention

As described above, in the prevention or treatment of stroke such as cerebral infarction and cerebral hemorrhage, particularly expansion of the infarct area, cerebral edema, and neurological symptoms in acute ischemic stroke, establishment of an effective and safer treatment is a definite medical need. Particularly, currently, from a viewpoint of safety of internal therapy and surgical operation therapy, there is a strong need of a safer pharmacotherapy which can be used over a long period. The present invention was done in view of such the backgrounds, and an object thereof is to provide a preventive or therapeutic agent for cerebral ischemic injury or cerebral ischemic reperfusion injury in stroke, which exhibits effectiveness by a different mechanism from that of the existing therapeutics, and can be taken over a long period.

### Means to Solve the Problems

Then, the present inventors evaluated (2S,4S)-6-fluoro-2',5'-dioxospiro[chroman-4,4'-imidazolidine]-2-carboxami de (generic name: Fidarestat) in order to show its effect on the cerebral ischemic injury or cerebral ischemic reperfusion injury using widely used mouse models with middle cerebral artery ischemia-reperfusion injury. As a result, it was found out that the drug has an effect on the neurological symptoms and the expansion of the cerebral infarction size which are observed in the mice with middle cerebral artery ischemia-reperfusion. That is, the present invention is a preventive or therapeutic agent for cerebral ischemic injury or cerebral ischemic reperfusion injury in stroke which contains 6-fluoro-2',5'-dioxospiro[chroman-4,4'-imidazolidine]-2-carboxamide (including racemate) as an active ingredient.

Specific examples of the stroke include cerebral infarction, cerebral hemorrhage, subarachnoid haemorrhage, and transient cerebral ischemia. Further, examples of the cerebral ischemic injury or cerebral ischemic reperfusion injury include those caused by thrombolytic therapy for acute ischemic stroke, those caused by a surgical intervention selected from the group consisting of hematoma evacuation, hematoma aspiration, and ventricular drainage, and those caused by carotid artery stent placement, bypass operation, or cervical carotid endarterectomy for cervical carotid artery occlusion or stenotic or obstructive lesion of an intracranial main artery.

Further, a preferable example of 6-fluoro-2',5'-dioxospiro[chroman-4,4'-imidazolidine]-2-carboxamide is optically resolved (2S,4S)-6-fluoro-2',5'-dioxospiro[chroman-4,4'-imidazolidine]-2-carboxami de (generic name: Fidarestat).

According to another aspect of the present invention, there is provided use of 6-fluoro-2',5'-dioxospiro[chroman-4,4'-imidazolidine]-2-carboxamide for manufacturing a preventive or therapeutic agent for cerebral ischemic injury or cerebral ischemic reperfusion injury in stroke. With reference to the matter, subordinate concepts of the present invention are established as with the present invention of the above-described agent.

### Effect of the invention

The present invention provides a preventive or therapeutic agent for cerebral ischemic injury or cerebral ischemic reperfusion injury in stroke such as cerebral infarction, cerebral hemorrhage, subarachnoid haemorrhage, and transient cerebral ischemia. Especially, when fidarestat is used as a drug, there is provided the safer pharmacotherapy which shows significant effects at low doses and can be administered over a long period.

### Brief Description of the Drawings

Fig. 1 shows the effect of fidarestat on the cerebral infarct size (therapeutic efficacy).
Fig. 2 shows the effect of fidarestat on the cerebral infarct size (protective effect).
Fig. 3 shows the effect on the cerebral infarct size of AR gene defects.
Fig. 4 shows the effect of fidarestat on the cerebral infarct volume (therapeutic efficacy).
Fig. 5 shows the effect of fidarestat on the cerebral infarct volume (protective effect).
Fig. 6 shows the effect on the cerebral infarct volume of AR gene defects.

### BEST MODE FOR CARRYING OUT THE INVENTION

Herein below, the present invention will be more specifically described.

The present invention is the preventive or therapeutic agent for cerebral ischemic injury or cerebral ischemic reperfusion injury in stroke which contains 6-fluoro-2',5'-dioxospiro[chroman-4,4'-imidazolidine]-2-carboxamide (including racemate) as an active ingredient. Specifically, examples of the stroke include cerebral infarction, cerebral hemorrhage, subarachnoid haemorrhage, and transient cerebral ischemia. Further, examples of the cerebral ischemic injury or cerebral ischemic reperfusion injury include those caused by thrombolytic therapy for acute ischemic stroke, those caused by a surgical intervention selected from the group consisting of hematoma evacuation, hematoma aspiration, and ventricular drainage, and those caused by carotid artery stent placement, bypass operation, or cervical carotid endarterectomy for cervical carotid artery occlusion or stenotic or obstructive lesion of an intracranial main artery. Among conditions produced by stroke, the preventive or therapeutic agent for cerebral ischemic injury or cerebral ischemic reperfusion injury in stroke of the present invention is effective, particularly in expansion of cerebral infarction, cerebral edema, core symptom, or neurologic deficit.

The present invention notes that all AR inhibitors may be used as the preventive or therapeutic agent for cerebral ischemic injury or cerebral ischemic reperfusion injury in stroke. Preferable examples of the AR inhibitor include hydantoin derivatives such as 6-fluoro-2',5'-dioxospiro[chroman-4,4'-imidazolidine]-2-carboxamide. Among them, optically resolved (2S,4S)-6-fluoro-2',5'-dioxospiro[chroman-4,4'-imidazolidine]-2-carboxami de (generic name: Fidarestat) is particularly preferable. Other examples of the AR inhibitor include Ranirestat (AS-3201), ARI-809, Epalrestat, Zopolrestat, Zenarestat, Tolrestat, Imirestat, Ponalrestat, Voglistat, TAT (WP-921), M-160209, SG-210, and NZ-314.

Although a protective agent for retinal or optic nerve in the present invention varies depending on the compound to be selected, the protective agent can be orally administered for example, as tablets, capsules, powders, granules, liquids or syrups, or can be parenterally administered as eye drops, injectables or suppositories, which were formed by conventional pharmaceutical manufacturing techniques. For pharmaceutical manufacturing, in the case of solid formulations, pharmaceutically acceptable excipients such as starch, lactose, purified white sugar, glucose, crystalline cellulose, carboxycellulose, carboxymethylcellulose, carboxyethylcellolose, calcium phosphate, magnesium stearate, gum arabic and the like can be used and, if necessary, lubricants, binders, disintegrating agents, coating agents, coloring agents and the like can be incorporated. In addition, in the case of liquid formulations, stabilizers, solubilizers, suspending agents, emulsifiers, buffers, preservatives and the like can be used. The dose is different depending on the compound being selected, symptoms, age, administration methods, dosage forms, and the like and, in the normal case, it is preferable that the preparation is administered to an adult in a range of 0.1 to 200mg, preferably 1 to 100mg per day in terms of the present compound for consecutive days, once or a few times a day. The above description is most suitable for, particularly the case where fidarestat is used.

### Examples

### 1. Test method

Mouse models with acute ischemic stroke, namely, mouse models with middle cerebral artery occlusion were used as evaluation systems. The experiment consists of three experiments, i.e., Experiment 1 to evaluate the therapeutic efficacy of fidarestat (control group and fidarestat administration group), Experiment 2 to evaluate the protective effect of fidarestat (control group and fidarestat administration group), and Experiment 3 to evaluate the role of AR (wild-type mouse group and AR gene-deficient mouse group).

Used mice (C57BL/6J line, 22 to 28 g of body weight) were subjected to right middle cerebral artery occlusion (MCAO) by the filament method under gas anesthesia over 2 hours. Thereafter, anesthesia was terminated and the mice were kept in an intensive care system (ThermoCare Inc) at 32°C for 4 to 6 hours. The neurological symptoms, the cerebral infarction size, and the cerebral infarction volume were used as end points.

The evaluation of the neurological symptoms was determined 22 hours after reperfusion based on the following four scores:
0: no neurological deficit (normal);
1: stretching of the opposite forefoot (mild);
2: contralateral circling (moderate); and
3: loss of walking and righting reflex (severe).

Evaluations of the cerebral infarction size and the cerebral infarction volume were performed as follows. After the evaluation of the neurological symptoms, the mouse brains were removed immediately and cut into 6 coronary slices with a thickness of 2 mm. In order to discover the sites of infarction, the slices were stained with 2% triphenyltetrazolium chloride (TTC) in a darkroom for 15 minutes and then fixed in 10% formalin buffer overnight. The posterior surface of each brain slice was photographed and analyzed using a digital image analyzing system (NeuroLucida, MicroBrightfield Inc.). The cerebral infarction size and the cerebral infarction volume (%) were calculated by an indirect method.

In Experiment 1 to evaluate the therapeutic efficacy of fidarestat, fidarestat was administered to the mice at 2 mg/kg 15 minutes before reperfusion. In Experiment 2 to evaluate the protective effect of fidarestat, fidarestat was administered to the mice at 10 mg/kg 30 minutes before occlusion. Only solvent was administered to the control group. In each case, forced intragastric administration was carried out.

### 2. Results

### (1) Effect on neurological symptoms

The results of Experiments 1 and 2 are shown in Tables 1 and 2. Fidarestat had a significant effect on the worsening of the neurological symptoms observed after ischemic reperfusion. Further, the results of Experiment 3 are shown in Table 3. Significant effects were observed in AR gene-deficient mice. The effect of fidarestat is similar to that of AR gene-defects.

**Table 1**

| Neurological symptoms | | | | | | |
|---|---|---|---|---|---|---|
| Distribution of neurological scores | | | | | | |
| Neurological scores | 0 | 1 | 2 | 3 | (Dead) | Mean±SEM |
| Vehicle (n=9) | 0 | 1 | 7 | 1 | (n=2) | 2.00±0.17 |
| Fidarestat (n=9) | 0 | 7 | 2 | 0 | (n=2) | 1.22±0.15* |

| | | | | | | |
|---|---|---|---|---|---|---|
| P<0.02 (Mann Whitney test) | | | | | | |

**Table 2**

| Neurological symptoms | | | | | |
|---|---|---|---|---|---|
| Distribution of neurological scores | | | | | |
| Neurological scores | 0 | 1 | 2 | 3 | Mean±SEM |
| Vehicle (n=9) | 0 | 2 | 7 | 0 | 1.77±0.14 |
| Fidarestat (n=9) | 0 | 7 | 2 | 0 | 1.22±0.14* |

| | | | | | |
|---|---|---|---|---|---|
| P<0.05 (Mann Whitney test) | | | | | |

**Table 3**

| Neurological symptoms | | | | | |
|---|---|---|---|---|---|
| Distribution of neurological scores | | | | | |
| Neurological scores | 0 | 1 | 2 | 3 | Mean±SEM |
| AR +/+(n=7) | 0 | 0 | 4 | 3 | 2.43±0.20 |
| AR -/-(n=8) | 0 | 5 | 3 | 0 | 1.38±0.18* |

| | | | | | |
|---|---|---|---|---|---|
| P<0.01 (Mann Whitney test) | | | | | |

### (2) Effects on the cerebral infarction size and the cerebral infarction volume

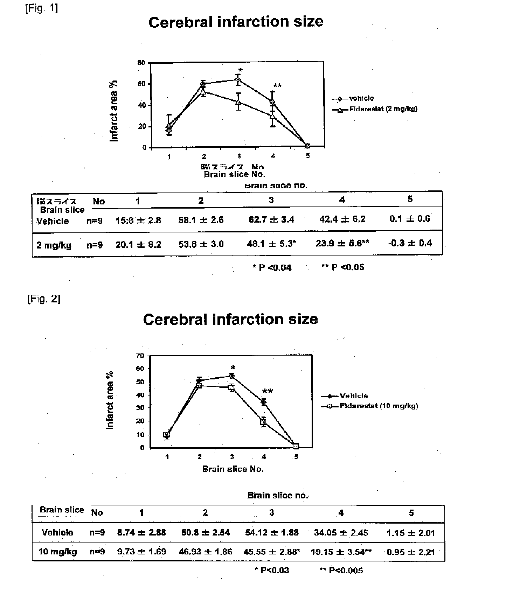

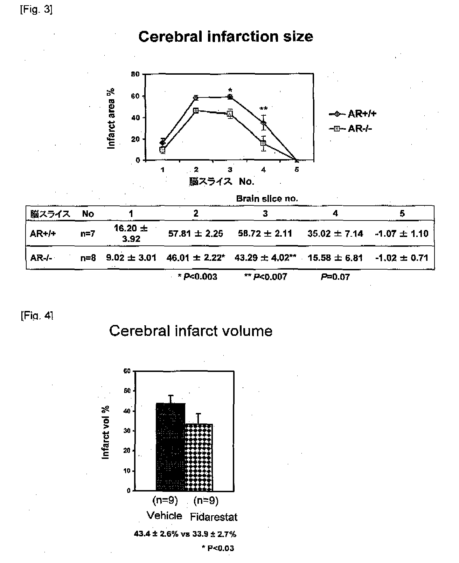

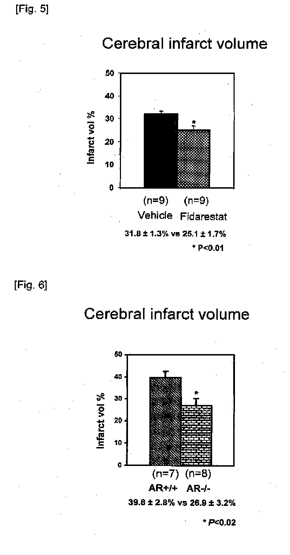

As the results of Experiments 1 and 2, fidarestat significantly reduced the cerebral infarction size observed after ischemic reperfusion (Figs. 1 and 2: brain slice Nos. 3 and 4). Additionally, significant effects were observed in AR gene-deficient mice of Experiment 3 (Figs. 3: brain slice Nos. 3 and 4). The effect of fidarestat is almost similar to that of AR gene-defects. In this regard, the same result was also obtained in the cerebral infarction volume (Figs. 4 to 6).

### 3. Discussion

It was confirmed that fidarestat exhibited effects on neurological symptoms, the cerebral infarction size, and the cerebral infarction volume which were observed after cerebral ischemic reperfusion in mouse models with middle cerebral artery occlusion. This shows that fidarestat can be used as the preventive or therapeutic agent for stroke such as cerebral infarction, cerebral hemorrhage, subarachnoid haemorrhage, and transient cerebral ischemia, particularly cerebral thrombosis in acute ischemic stroke or the worsening of neurological symptoms due to cerebral infarction and expansion of the infarct area. Further, the ischemic reperfusion model exhibited the same condition as that caused by thrombolytic therapy for acute phase cerebral infarction. It is apparent that fidarestat is effective in preventing and treating exacerbation of cerebral infarction, i.e., reperfusion injury caused by revascularization using a thrombolytic therapy drug.

Furthermore, in AR gene-deficient mice, inhibitory effects on the worsening of neurological symptoms and the expansion of the cerebral infarction size and the cerebral infarction volume were observed and they were similar to that of fidarestat. From the experimental facts, it is found that these effects are effects of AR inhibition. In other words, it is found out that other AR inhibitors are also effective in preventing or treating cerebral ischemic injury or cerebral ischemic reperfusion injury.

## Claims

1. A preventive or therapeutic agent for cerebral ischemic injury or cerebral ischemic reperfusion injury in stroke, comprising 6-fluoro-2',5'-dioxospiro[chroman-4,4'-imidazolidine]-2-carboxamide as an active ingredient.

2. The preventive or therapeutic agent according to claim 1, wherein the stroke is cerebral infarction, cerebral hemorrhage, subarachnoid haemorrhage, or transient cerebral ischemia.

3. The preventive or therapeutic agent according to claim 1, wherein the cerebral ischemic injury or cerebral ischemic reperfusion injury is caused by thrombolytic therapy for acute ischemic stroke.

4. The preventive or therapeutic agent according to claim 1, wherein the cerebral ischemic injury or cerebral ischemic reperfusion injury is caused by a surgical intervention selected from the group consisting of hematoma evacuation, hematoma aspiration, and ventricular drainage.

5. The preventive or therapeutic agent according to claim 1, wherein the cerebral ischemic injury or cerebral ischemic reperfusion injury is caused by carotid artery stent placement, bypass operation, or cervical carotid endarterectomy for cervical carotid artery occlusion or stenotic or obstructive lesion of an intracranial main artery.

6. The preventive or therapeutic agent according to any of claims 1 to 5, wherein the 6-fluoro-2',5'-dioxospiro[chroman-4,4'-imidazolidine]-2-carboxamide is (2S,4S)-6-fluoro-2',5'-dioxospiro[chroman-4,4'-imidazolidine]-2-carboxami de (generic name: Fidarestat).

7. Use of 6-fluoro-2',5'-dioxospiro[chroman-4,4'-imidazolidine]-2-carboxamide for the manufacture of a preventive or therapeutic agent for cerebral ischemic injury or cerebral ischemic reperfusion injury in stroke.

8. The use of 6-fluoro-2',5'-dioxospiro[chroman-4,4'-imidazolidine]-2-carboxamide according to claim 7, wherein the stroke is cerebral infarction, cerebral hemorrhage, subarachnoid haemorrhage, or transient cerebral ischemia.

9. The use of 6-fluoro-2',5'-dioxospiro[chroman-4,4'-imidazolidine]-2-carboxamide according to claim 7, wherein the cerebral ischemic injury or cerebral ischemic reperfusion injury is the one caused by thrombolytic therapy for acute ischemic stroke.

10. The use of 6-fluoro-2',5'-dioxospiro[chroman-4,4'-imidazolidine]-2-carboxamide according to claim 7, wherein the cerebral ischemic injury or cerebral ischemic reperfusion injury is the one caused by a surgical intervention selected from the group consisting of hematoma evacuation, hematoma aspiration, and ventricular drainage.

11. The use of 6-fluoro-2',5'-dioxospiro[chroman-4,4'-imidazolidine]-2-carboxamide according to claim 7, wherein the cerebral ischemic injury or cerebral ischemic reperfusion injury is the one caused by carotid artery stent placement, bypass operation, or cervical carotid endarterectomy for cervical carotid artery occlusion or stenotic or obstructive lesion of an intracranial main artery.

12. The use of 6-fluoro-2',5'-dioxospiro[chroman-4,4'-imidazolidine]-2-carboxamide according to any of claims 7 to 11, wherein the 6-fluoro-2',5'-dioxospiro[chroman-4,4'-imidazolidine]-2-carboxamide is (2S,4S)-6-fluoro-2',5'-dioxospiro[chroman-4,4'-imidazolidine]-2-carboxami de (generic name: Fidarestat).
